# EUROPEAN PATENT APPLICATION

(11) **EP 4 848 258 A2**
(43) Date of publication of application: **02.12.2026**
(21) Application number: 26212882.0
(22) Date of filing: 12.04.2024
(51) Int. Cl.: G09B 23/28

(54) **SURGERY SIMULATION SYSTEM AND METHOD**

(30) Priority: 14.02.2023 US 202318109491
(62) Document number(s) of the earlier application(s) in accordance with Art. 76 EPC: 24721758.1 / 4 666 274
(71) Applicant: Papadonikolakis, Anastasios, Winston Salem, North Carolina 27104 (US); Papadonikolakis, Pavlos, La Mesa, California 91942 (US)
(72) Inventor: Papadonikolakis, Anastasios, Winston Salem, North Carolina 27104 (US); Papadonikolakis, Pavlos, La Mesa, California 91942 (US)
(74) Representative: Araujo, Daniel

(57) **Abstract**

A surgery simulation systemincludes an anatomical model with one or more apertures, at least one surgical tool having a part which is movable though the one or more apertures, a first virtual reality system having a computer system and a set of tracking sensors, and a second virtual reality system that includes the computer system and one or more proximity sensors which are located at the one or more apertures. The computer system is configured to generate a first virtual reality model and a second virtual reality model. A method for simulating a surgery is also disclosed.

## Description

### BACKGROUND

### Field

The present disclosure concerns a surgery simulation system and a related method. It particularly concerns simulations systems which are based on the use of virtual reality. Such systems can be used for simulating a surgical procedure, or at least parts of a surgical procedure. Also, such a system may be called surgery simulation apparatus or surgery simulator.

### Backround Information

There are known surgery simulation systems in which the user of the system physically executes actions that simulate completely or partly a surgical procedure, and simultaneously the user sees on a screen a virtual reality model that simulates in real time the actions executed by the user. Typically, such surgery simulation systems involve tracking the position of some physical objects with which the user interacts, and also involve one or more computers for generating the virtual reality model according to the tracked position. For example, some surgical simulators are described in US2022/139260 A1, which utilizes electromagnetic motors for axial haptic feedback, US2022/101745 A1, focused on virtual reality environments for robotic surgery simulation, and US2016/314710 A1, which implements another virtual reality surgery simulation system. An important problem with the previously known systems is that the accuracy of said tracking, i.e. the accuracy with which the position of the objects is tracked by the system, is very poor. Consequently, in these previously known systems there may be significant deviation, i.e. discrepancy, between the real position of the real objects, and the position of the virtual representations of said objects in the virtual reality model seen by the user on the screen. Such a positional deviation (positional discrepancy) is particularly problematic considering that in a surgical procedure, the actions taken by the medical staff which executes the procedure must generally be precise and of a high positional accuracy. Hence, a surgery simulation system for being educationally and professionally useful to its users, as well as for being pleasant to use, must offer to its users a realistic experience which includes high tracking accuracy of the user's actions, so that the virtual model shown to the user is also correct and accurate.

For overcoming the above problem there have been previously proposed systems in which the virtual reality model being shown to the user is being corrected, for example, by transforming (altering) the surfaces of the representations of the objects in the virtual model, or by applying corrections to the virtual model that is generated. Nevertheless, such previously known approaches are complex to implement, and instead of trying to solve the problem of how to generate correct virtual models, they simply try to bypass the problem by correcting intrinsically wrong virtual models. The present invention solves these problems.

### SUMMARY

The present invention offers a virtual reality system and a related method that can provide high tracking accuracy and a correct virtual reality model. Consequently, the present invention can provide a realistic, useful and pleasant experience which can greatly help medical staff in the planning and training before a real surgical procedure. Advantageously, the present invention is easy and simple to implement, without being excessively complex.

The invention in its first aspect concerns a surgery simulation system which comprises an anatomical model, at least one surgical tool, a first virtual reality system and a second virtual reality system. The anatomical model has (i.e. comprises) one or more apertures. The least one surgical tool comprises a part which is movable though the one or more apertures. The first virtual reality system comprises a computer system and a set of tracking sensors which are on the anatomical model and on the at least one surgical tool. The set of tracking sensors are connectable to the computer system and configured for tracking respective positions of the anatomical model and of the at least one surgical tool. Also, the computer system is configured to define first coordinates of the anatomical model and of the at least one surgical tool according to the tracked positions. The second virtual reality system comprises the computer system and one or more proximity sensors. The proximity sensors are located at the one or more apertures. Each of the one or more proximity sensors is configured to detect an insertion of the part of the at least one surgical tool at the respective aperture where the proximity sensor is located. When said insertion is detected, then the computer system is configured to define second coordinates of the anatomical model and of the inserted surgical tool. The second coordinates of the inserted surgical tool are a function of the second coordinates of the anatomical model. Also, the computer system is further configured to generate a first virtual reality model according to the first coordinates. Also, when the computer defines the second coordinates, the computer is configured to generate a second virtual reality model according to the second coordinates. The first virtual reality model comprises respective virtual representations, i.e. first virtual representations, of the anatomical model and of the at least one surgical tool. The second virtual reality model comprises respective virtual representations, i.e. second virtual representations, of the anatomical model and of the inserted surgical tool.

The anatomical model simulates a part of the body on which the real surgical operation would be performed. The anatomical model may be made of plastic, or other materials, or of a plurality of materials. The apertures on said model may be openings, holes or orifices on a surface of the anatomical model, and may simulate incisions made to said body in a hypothetical real operation, or may represent naturally occurring opening on or within said body. There may be more than one apertures (openings) on the anatomical model, corresponding to more than one corresponding openings found in the body which would be operated in a hypothetical real surgical procedure. If there are more than one openings, these may allow for the simultaneous insertion of more than one surgical instruments into the body. For example, said openings may allow for the simultaneous insertion of one surgical tool per opening, because a real surgical procedure could involve inserting more than one surgical instruments in the operated body. In preferred embodiments of the invention, the anatomical model is a model of a shoulder, of a shoulder joint or of another part of a human or animal body.

The surgical instruments used in the surgical simulation system of the first aspect of the invention, may be real surgical instruments or they may be models or replicas of real surgical instruments which may or may not comprise all the parts found in the corresponding real surgical instruments. Likewise, said surgical instruments comprised in the surgical simulation system, may be made of the same materials as real surgical instruments, or they be made of different materials. In addition, said surgical instruments when compared to real corresponding surgical instruments may comprise additional parts, such as additional electronic components.

Nevertheless, it is preferred that the surgical instruments are identical to real ones, or similar to real ones, so that their use during the surgery simulation contributes to an experience which is as realistic as possible. The surgical instruments may also be called surgical tools and *vice versa*.

As mentioned above, the surgical simulation system may optionally comprise a plurality of surgical tools. These may be used for simulating different surgical operations, some of which may involve using more than one surgical tools. For this reason, in some preferred embodiments of the first aspect of the invention, the surgery simulation system comprises two, three or more surgical tools, preferably the surgical tools being any of an arthroscope, an electrocautery, a grasper, a suture passer or a suture handling device. Some or all of the aforementioned surgical tools may comprise blunt tips.

Moreover, in a preferred embodiment, the at least one surgical tool is an electronic one with a button and/or a handle, and is connectable to the computer system such that when the button and/or handle is/are operated by a user, the computer system includes in the second virtual reality model a virtual effect that represents a virtual surgical action which is executable by the virtual representation of the respective surgical tool. Therefore, if for example the surgical tool is an electrocautery, it may have a button or handle which when pressed or actuated by the user, it may trigger the computer so that the latter simulates in the second, or even the first, reality model a virtual electrocautery effect. It may be understood than in the latter case the surgical tool may have an electronic system which is configured for operationally communicating, wirelessly or via wire, with the computer system, so that it emits to the system a signal when said button or handle is actuated. Said signal when received by the computer may trigger the computer to simulate said virtual surgical action.

The computer system of the first virtual reality system may be a personal or professional computer, or may be a mobile computer, or may be a remote computer which is connected to the sensors wirelessly or via wires or via the internet, or may comprise more than one computers. Computers and computer systems for virtual reality applications are known in the art. The computer system is part of both the first and second virtual reality systems. However, the computer system may comprise a first computer system or module that is comprised by the first virtual reality system and is connected to the tracking sensors, and a second computer system or module that is comprised by the second virtual reality system, wherein the first and the second computer systems are operationally connected to each other.

The set of tracking sensors are connectable to the computer system, and hence, the computer system may receive and process signals from the tracking sensors. The computer system may operate in conjunction with the tracking sensors for tracking and registering the tracked positions of the anatomical model and of the at least one surgical tool. The tracked positions can be registered on a memory of the computer system, and the respective information may be used for the generation of the first and/or second virtual reality model(s). In a preferred embodiment there is a tracking sensor on the anatomical model, and there is also one tracking sensor on each surgical tool used in the surgical simulation system. Preferably, on each surgical instrument there is a respective one tracking sensor located on an end (extreme) of the surgical instrument which is opposite to the surgical instrument's part that forms the other end of the surgical instrument. Hence, when said part is inserted through one opening of the anatomical model, the tracking sensor on the surgical instrument may remain outside the anatomical model.

In a preferred embodiment, the tracking sensors and/or the computer system may be parts of a commercially available virtual reality system or motion capture system that can be generally used for virtual reality applications. Moreover, such a commercially available virtual reality system, as well as the surgery simulation system of the invention, may comprise additional components which may, together with the tracking sensors, form a tracking system for virtual reality applications, i.e. a virtual reality tracking system. For this reason, in a preferred embodiment of the invention, the surgery simulation system comprises a virtual reality tracking system, and the tracking sensors and/or a part of the computer system are parts of said tracking system. Similarly, in a preferred embodiment, the first virtual system comprises a tracking system which comprises the tracking sensors, and said tracking system is a virtual reality tracking system. For example, such a tracking system may comprise a set of beacons which emit optical or other type of electromagnetic signals, and the tracking sensors may operate by receiving the signals emitted by the beacons of the tracking system. Said signals may be processed and compared by the tracking sensors, or by a computer of the tracking system, or by the computer system of the first virtual reality system, for therefore identifying the position of the tracking sensor with respect to the beacons. Also, in a preferred embodiment the computer system of the first virtual reality system comprises a computer subsystem or a computer module that is part of the aforementioned tracking system.

The definition of the first coordinates allows for the generation of the first virtual reality model.

The main purpose of the first virtual reality model is to simulate or depict the procedure happening when the surgical instrument is outside the anatomical model i.e. when the surgical instrument's part has not been inserted yet through or in the at least one aperture of the anatomical model. It is useful to simulate said process even when the surgical instrument is still outside the anatomical model, because that may give the opportunity to the user of the surgery simulation system to become familiar with the system and its components, and also become familiar with holding and handling the at least one surgical instrument around the anatomical model. In addition, the around the anatomical model interaction of the user with the surgical instruments may be crucial because it may allow for simulation of parts of a surgical procedure such as passage of a suture through a wire loop, loading of a drill or any other preparation for the execution of a surgical action within the anatomical model. Moreover, if the virtual model is shown to the user via a display, as is described further below, the first virtual model can help the user to become familiar with the (first) virtual representations of the surgical instrument and of the anatomical model.

The second virtual system serves the purpose of enabling the creation of a second virtual reality model in which the positions of the inserted surgical tool with respect to the anatomical model is very accurate. This is achieved by the fact that the second coordinates of the surgical tool are defined as a function of the second coordinates of the anatomical model. Therefore, the coordinates of the anatomical model as a whole, or of a specific point of the anatomical model may in effect act as a reference point for defining, and therefore, tracking, the position of the inserted surgical instrument with respect to the surgical model. Preferably, the second coordinates of the surgical instrument are a function of the second coordinates of the opening, e.g. a point at said opening, in which the surgical instrument is inserted. Given that, once the surgical instrument is inserted in the opening, the movement of the surgical instrument is physically restricted by the opening´s physical borders (walls), the degrees of freedom of the surgical instrument's movement may in effect be reduced or restricted. The second virtual reality system takes advantage of said apparent restriction imposed on how the inserted surgical instrument can move through the opening, and in effect can use it for accurately defining second coordinates of the inserted surgical instrument as a function, e.g. with respect to, the coordinates of the anatomical model.

The proximity sensors are used for detecting the insertion of the at least one surgical instrument though the one or more apertures of the anatomical model, and therefore, said proximity sensors, when they sense or detect the presence or insertion of the surgical instrument in the opening, may trigger the computer system to start defining the second coordinates and to generate the second virtual reality model. The proximity sensors may preferably be capacitive sensors, or optical sensors, or other types of sensors, and may be connected to the computer system directly or indirectly, e.g. via an electronic interface.

The first virtual reality model serves the purpose of simulating the handling of the at least one surgical instrument while the latter is outside the anatomical model. The first virtual representations of the anatomical model and of the surgical instrument may be computer graphics or respective data, and permit the possible creation of a virtual reality view, i.e. of an image or video, which is realistic and shows the positioning and motion of the surgical instrument and anatomical model in a virtual space or environment. The relative positioning of the first virtual representations of the model and of the surgical tool with respect to each other, may not accurately represent the relative positioning of the (physical) surgical instrument and model, nevertheless, it is preferred that the first virtual representations themselves are as realistic as possible, e.g. very similar or identical to the form and appearance of the (physical or real) surgical instrument and model.

Once the surgical instrument's part, which may be a bland tip or shaft of the surgical instrument, is inserted in the opening, then the second virtual reality system enables the creation of the second virtual reality model, which as mentioned further above, can represent accurately the relative positioning of the inserted surgical instrument with respect to the anatomical model. Similarly to what is described further above regarding the first virtual representations, preferably the second virtual representations of the inserted surgical instrument and of the anatomical model are as realistic as possible e.g. very similar or identical to the form and appearance of the (physical) surgical instrument and model. Therefore, the first virtual representation of the anatomical model may be very similar or identical to the second virtual representation of the anatomical model. Likewise, the second virtual representation of the surgical instrument may be very similar or identical to the second virtual representation of the surgical instrument. It can be understood that optionally the first and second virtual models can comprise more virtual representations of more physical objects which may be additional optional parts of the surgery simulation system. In an example, the surgery simulation system may comprise a platform on which there is fixed the anatomical model, and in said example, the first and second virtual models comprise respective virtual representations of said platform.

In a preferred embodiment according to first aspect of the invention, the surgery simulation system further comprises a cart on which there is the anatomical model. Such a cart can act as the aforementioned optional platform on which the anatomical model may be. Said cart may support more parts of the surgery simulation system, for example may support or carry the computer system, the at least one surgical tool, and/or an optional display as the one described further below.

In a preferred embodiment according to first aspect of the invention, the surgery simulation system further comprises a movable platform, and the anatomical model is on the movable platform which is configured for controllably rotating and/or linearly moving the anatomical model. Said movable platform may be on the aforementioned optional cart, and its motion and/or rotation may advantageously allow for the user positioning the anatomical model before or during the surgery simulation.

In a preferred embodiment according to first aspect of the invention, the computer system is further configured to define the second coordinates of the inserted surgical tool as a function of the first coordinates of the inserted surgical tool. In that case, the second coordinates of the inserted surgical tool would be a function of the second coordinates of the anatomical model and of the first coordinates of the inserted surgical tool. Therefore, it can be understood that when the second virtual reality system defines the second coordinates, the first virtual reality system may continue defining the first coordinates of the inserted surgical instrument.

Advantageously, this enables using the first virtual reality system, which may be a system that as such offers limited or poor positional tracking accuracy, as a part of the second virtual reality system which offers very high positional accuracy. For the above reasons, in a preferred embodiment according to first aspect of the invention, the computer system is configured to define and store in a memory of it the first coordinates while it defines the second coordinates. In a preferred embodiment according to first aspect of the invention, the second coordinates of the anatomical models are second coordinates of the aperture in which there is the inserted surgical tool. In that case, the second coordinates of the inserted surgical tool would be a function of the second coordinates of the aperture, or of a point at or about said aperture, in which the surgical instrument is inserted. This can permit tracking the inserted surgical instrument using the aperture as a reference (reference point), so that said tracking can give accurate information regarding the position of the inserted surgical instrument with respect to opening in which the surgical instrument is inserted.

In a preferred embodiment according to first aspect of the invention, the second coordinates of the anatomical model are fixed i.e. do not vary during the generation of the second virtual model. Hence, the values of the second coordinates may be fixed. Advantageously, this can limit the computational power required for the generation of the second virtual model. In a preferred embodiment, the second coordinates of a point of the anatomical model, e.g. of a point of the hole in which the surgical instrument is inserted, are fixed as zero (e.g. x=0, y=0, z=0) and the second coordinates of the inserted surgical tool are a function of, e.g. defined with respect to, said zero coordinates.

In a preferred embodiment according to the first aspect of the invention, the computer system further defines the second coordinates of the inserted surgical tool as a function of a length of the inserted surgical tool. Hence, the second coordinates of the inserted surgical tool can be a function of both the length of the surgical tool and the second coordinates of the anatomical model. In an example, the tracking sensor on the inserted surgical tool is located at the end of the surgical tool that is opposite to the tool's end that first enters in the opening. Therefore, in said example, at the beginning of the tool's insertion it can be known that the distance of the tracking sensor from the anatomical model's opening is equal to the surgical tool's length. This information can be used for defining accurately initial values of the second coordinates of the surgical tool at the moment it starts its insertion, i.e. at the moment said insertion is detected by the proximity sensor, and said information can be further be used for defining also accurately subsequent values of the second coordinates of the surgical tool during the latter's movement through the opening.

In a preferred embodiment according to first aspect of the invention, the surgery simulation system further comprises a display which is connectable to the computer system which is configured to render on the display the first and the second virtual reality model. More preferably, the display is a computer monitor. Most preferably, the surgery simulation system also comprises a virtual reality headset connectable to the computer system, and the computer system is configured to render the first and second reality models on both, or either of, the computer monitor and the virtual reality headset.

In a preferred embodiment according to first aspect of the invention, the surgery simulation system comprises the display as described further above, the computer system is configured to render on the display the first and the second virtual reality model as described further above, and moreover, when the computer system defines the second coordinates, it is configured to then stop rendering on the display the first virtual reality model, and to start rendering on the display the second virtual reality model.

The skilled person can understand that similarly to what often happens in real surgical procedures, the surgical tool when moving through the anatomical model's opening may effectively execute a piston and/or a pivot movement. This may be happening due to the actual physical dimensions of the opening, and the morphology of the anatomical model which may restrict the type of the movement that the surgical tool can follow once being inserted.

Accordingly, in a preferred embodiment which is according to the first aspect of the invention, for generating the second virtual model, the computer system is further configured to calculate parameters of a piston movement and/or a pivot movement of the inserted surgical tool about the corresponding aperture at which there is the inserted surgical tool. Said parameters may be a pivot angle, or a displacement along an axis of movement.

The part of the surgical instrument which is insertable via said aperture (opening) of the anatomical model, may be a substantially elongated one, and in a preferred embodiment is a shaft. It is contemplated that the system of the first aspect of the invention is particularly useful for simulating the use of an arthroscope. There are known types of surgical arthroscopes which have a particularly elongated form. Accordingly, in a preferred embodiment that is according to an aspect of the invention, the at least one surgical tool is an arthroscope or endoscope or a surgical tool that has a camera on it.

In a preferred embodiment according to first aspect of the invention, the part (i.e. the part that is insertable through the opening) of the at least one surgical tool comprises a tip and a camera at or behind said tip or optically coupled to said tip. More preferably, the first and/or the second virtual reality models comprise a virtual camera corresponding to the camera at said tip.

Cameras are often used on surgical tools and or surgical operations, for example an arthroscope with a tip may have a camera which may be on or behind said tip, or may be optically coupled/connected to said tip, so that when the arthroscope's tip is inserted in a body, the camera can provide image or video from within the body. For the same reason, the instrument may comprise an optical configuration, e.g. a configuration that comprises one or more lenses, for transferring an optical signal from the tip to the camera. Likewise, the surgical instrument with the camera may also comprises an illumination system, e.g. a LED, which illuminates the region that is monitored with the camera. Hence, in the aforementioned preferred embodiment, the camera on the surgical tool may be used for providing a view (image or video) from within the anatomical model. When the surgical simulation system also comprises a display, the view taken from the camera can be displayed via said display together with, e.g. side by side, with the first and/or second virtual reality model, or can be overlaid or embedded within said virtual reality models. Moreover, the computer may be configured for generating and displaying a virtual reality camera view which may be similar or different to the view taken by the camera found on the surgical tool. Moreover, although the surgical tool may comprise the aforementioned optional camera, the latter may not be operated during the procedure, and the computer system may be configured to generate a virtual reality camera view that simulates a view taken by the camera if the latter was in operation. Also, in a preferred embodiment wherein the surgical instrument comprises the optional camera as described further above, the first and/or the second virtual reality model comprises a virtual camera view simulating an imaging by the camera at said tip. Also, in a preferred embodiment, the first and/or the second virtual reality model comprises a virtual camera view that simulating an imaging by a camera, regardless of whether the surgical instrument comprises or not said camera.

As mentioned further above, the present invention allows for tracking and defining the coordinates of the inserted surgical tool, with a very high accuracy which may be superior to the one achieved with previously known systems. Therefore, while previously known systems could offer tracking with an error in the range of several centimeters, which is a very poor tracking accuracy for simulating surgeries, with the system according to the first aspect of the invention, it is possible to achieve a much better accuracy. Advantageously, with the system of the invention, the error between the position of the inserted surgical tools with respect to the anatomical model, and the respective position that is defined from the second coordinates, can be as little as few millimeters. Hence, in preferred embodiment of the first aspect of the invention, the second virtual system is configured for tracking, with an error of 3 mm or less, the relative position of the inserted surgical tool with respect to the anatomical model.

In a preferred embodiment according to first aspect of the invention, the at one least surgical tool comprises a first inertial measurement unit, and the tracking sensor on the at least one surgical tool comprises a second inertial measurement unit, wherein the first and second inertial measurement units are configured to measure respective rotations of the surgical instrument and of the tracking sensor, and optionally and preferably to operationally emit to the computer system signals related to their measurements. More preferably, the first inertial measurement unit is at a front part or shaft of the surgical instrument, the tracking sensor with the second inertial measurement unit is at a rear part of the surgical instrument, and the computer system is configured to receive signals related to measurements taken from the first and second inertial measurement units, and to calculate a rotation and/or displacement of the surgical instrument.

The present invention in its second aspect concerns a method for simulating a surgery, and said method comprises: providing an anatomical model with one or more apertures, at least one surgical tool that comprises a part which is movable though the one or more apertures, a display which is connectable to a computer system, a first virtual reality system that comprises the computer and a set of tracking sensors on the anatomical model and on the surgical tool, and a second virtual reality system that comprises the computer system and one or more proximity sensors which are located at the one or more apertures, the tracking sensors being connected to the computer system; with the tracking sensors tracking respective positions of the anatomical model and of the surgical tool; with the computer system (i.e. using the computer system) defining first coordinates of the anatomical model and of the at least one surgical tool according to their respective tracked positions; with the one or more proximity sensors detecting an insertion of the part of the at least one surgical tool at the respective aperture where the proximity sensor is located; with the computer system defining second coordinates of the anatomical model; with the computer system defining second coordinates of the inserted surgical tool as a function of the second coordinates of the anatomical model; with the computer generating a first virtual reality model according to the first coordinates, the first virtual reality model comprising virtual representations of the anatomical model and of the at least one surgical tool; with the computer system generating a second virtual reality model according to the second coordinates, the second virtual reality model comprising respective virtual representations of the anatomical model and of the inserted surgical tool.

In a preferred embodiment of the method according to the second aspect of the invention, the computer defines the second coordinates of the inserted surgical tool with respect to (e.g. referenced to) the second coordinates of the anatomical model, and said second coordinates of the anatomical model have fixed values.

A third aspect of the present invention concerns a surgery simulation system which is the same as the surgery simulation system of the first aspect of the invention, but with the following difference: it does not comprise the tracking sensors. Hence, the surgery simulation system according to the third aspect of the invention comprises the anatomical model, the least one surgical tool, the computer system and the second virtual reality system. The anatomical model and the at least one surgical tool are configured to receive on them the tracking sensors which are described further above. Also, the computer system is configured to be connected with the tracking sensors or a tracking system, e.g. a virtual reality tracking system, that comprises said tracking sensors, so that the first virtual reality system is formed once the computer system is operationally connected with (e.g. can receive signals from) said tracking sensors or tracking system. Given that there are commercially available virtual reality tracking systems, the surgery simulation system of the third aspect of the invention allows for using said commercially available tracking systems for achieving high quality surgery simulations. Any feature of the embodiments of the first aspect of the invention described herein, can be also found in corresponding embodiments of the second or third aspect of the invention, and *vice versa*.

### BRIEF DESCRIPTION OF THE DRAWINGS

FIG. 1 shows an embodiment of the surgery simulation system of the first aspect of the invention, with one surgical tool being inserted in the anatomical model.
FIG 2 shows the embodiment of FIG. 1 from a different perspective, but with the surgical tool being outside the anatomical model.
FIG. 3 shows the embodiment of FIG. 2 from a different perspective.
FIG. 4 shows an embodiment that includes the system of FIG. 2 and further includes additional components.
FIG. 5 shows an anatomical model of an embodiment of the invention.
FIG. 6 shows is a flow diagram of an embodiment of the method of the second aspect of the invention.
FIG. 7 shows is a flow diagram of a computer program in the computer system of an embodiment of the invention.

### DETAILED DESCRIPTION

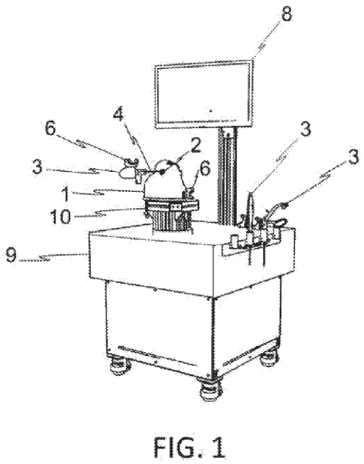

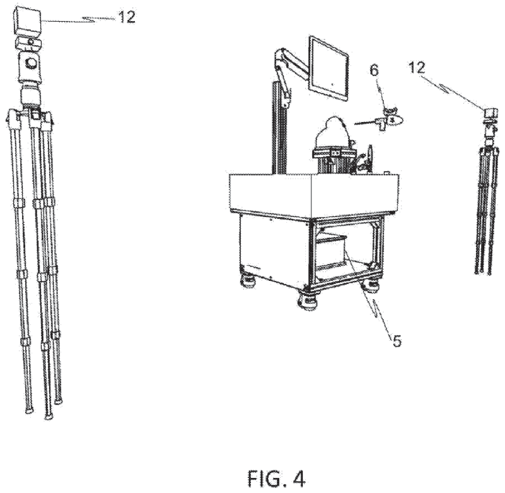

FIG. 1-4 show a surgery simulation system, comprising: an anatomical model 1 with one or more apertures 2; at least one surgical tool 3 that comprises a part 4 which is movable though the one or more apertures 2; a first virtual reality system that comprises a computer system 5 (FIG. 4) and a set of tracking sensors 6 which are on the anatomical model 1 and on the at least one surgical tool 3, the set of tracking sensors 6 being connectable to the computer system 5 and configured for tracking respective positions of the anatomical model 1 and of the at least one surgical tool 3, and wherein the computer system 5 is configured to define first coordinates of the anatomical model 1 and of the at least one surgical tool 3 according to the tracked positions; a second virtual reality system that comprises the computer system 5 and one or more proximity sensors 7 which are located at the one or more apertures 2, and each of the one or more proximity sensors 7 is configured to detect an insertion of the part 4 of the at least one surgical tool 3 at the respective aperture 2 where the proximity sensor 7 is located, and when said insertion is detected, then the computer system 5 is configured to define second coordinates of the anatomical model 1 and of the inserted surgical tool, the second coordinates of the inserted surgical tool being a function of the second coordinates of the anatomical model; wherein the computer system 5 is further configured to generate a first virtual reality model according to the first coordinates, and when the computer system 5 defines the second coordinates, the computer system 5 is configured to generate a second virtual reality model according to the second coordinates, the first virtual reality model comprising respective virtual representations of the anatomical model 1 and of the at least one surgical tool 3, and the second virtual reality model comprising respective virtual representations of the anatomical model 1 and of the inserted surgical tool.

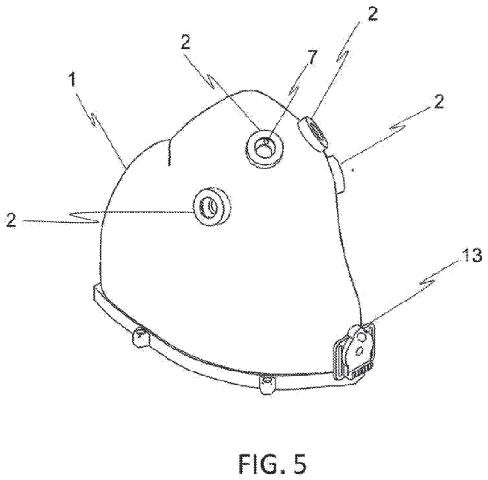

The computer system 5 of the system of FIG. 1-4 is a personal computer (pc) appropriately configured as described above. In the embodiment shown in FIG. 1-4 the surgery simulation system further comprises a display 8 connected to the computer 5 and a cart 9. On the cart 9 there is the anatomical model which in the shown preferred embodiment is a 3D printed model of a shoulder and is on a rotating mechanism which is manual. Said computer system 5 is within the cart 9 and is shown in FIG. 4 wherein some side panels are removed from the cart 9 so that the computer 5 is visible. The anatomical model of FIG. 1 is further shown in FIG. 5 that also shows that said model comprises a slot 13 one which one of the tracking sensors 6 can be attached on the anatomical model. The surgical tools 3 may also comprise similar slots for attaching thereat corresponding sensors 6. The embodiment shown in FIG. 1 further comprises mechanisms 10 which allow for change of the orientation or position of the shoulder model (i.e. the anatomical model). In the embodiment of FIG. 1 the position of the model (anatomical model) is tracked by the computer unit (the computer system). The aforementioned mechanism comprises tightening nuts which allow for vertical rotation. The mechanism for horizontal spinning of the pedestal (platform) is located inside the cart. There are also locking mechanisms with shear spring plungers every 45 degrees or rotation. The shoulder model- grey-has the apertures (holes) 2 for the insertion of the surgical instruments (surgical tools) 3 in the shoulder joint - inside the plastic model.

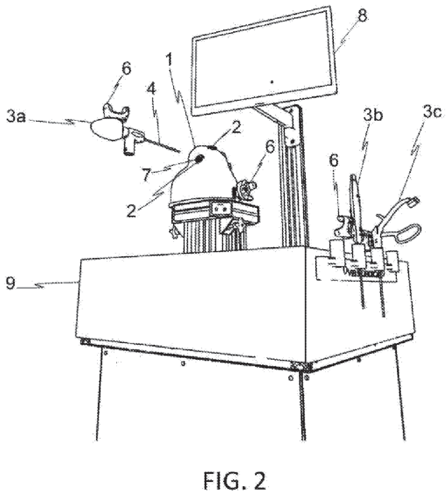

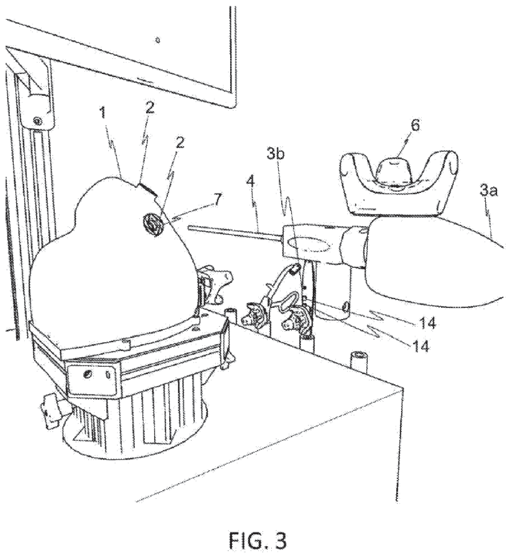

FIG. 1-4 show the surgical instruments used in a preferred embodiment of the system according to the third aspect of the invention. The surgery simulation system according to the third aspect of the invention may not not comprise the tracking sensors, hence the surgical instruments may be be configured, e.g. may have slots, for such tracking sensors being attached on the surgical instruments/slots. As shows in FIG. 1-3, the surgical tools 3, i.e. the surgical instruments, when not handled by the user they can be attached on the side of the cart. As shown in FIG. 2, the surgical instruments 3 of the embodiment of FIG. 1-4 are an electronic arthroscope 3a, an electronic electrocautery 3b and an electronic grasper 3c and have blunt tips. The tip of each of the shown instruments 3a, 3b, 3c is located at the end of the corresponding part 4 of each instrument. As shown in FIG. 3, the electronic electrocautery 3b has two buttons 14 for submission of signals of on and off to the computer system to simulate burning of tissue. The computer system may also be called processing unit. The grasper 3c has a sensor on the rear handle that uses electromagnetic field to simulate closure of teeth of the grasper in the virtual environment (first or second virtual model).

FIG. 2-3 also show an arthroscope 3a with a tracking sensor 6 mounted on it. The arthroscope of FIG. 3 can be used with the system of FIG. 1-4 so that the user can insert the arthroscope (a shaft of the arthroscope) in the plastic shoulder model 1 through the holes 2, so that arthroscopy virtual images are seen on the monitor (display) of the cart, or in a headset, in a 3D virtual environment. The tracking of the movement of the surgical instruments in the plastic shoulder model can be done with the tracking sensors 6, also called trackers, which in a preferred embodiment shown in FIG. 4 are part of a commercially available virtual reality tracking system which also comprises lighthouses 12 i.e. beacons. With reference to FIG. 1-4, tracking using said virtual reality tracking system may be done as follows. The trackers 6 are mounted on the surgical instruments 3. The user may have the option to select between 3D Virtual Reality display or 2D display on the monitor 8 that is located on the cart 9. With the aid of the lighthouses and the truckers, the position of the instrument in the anatomical model 1 is tracked as the user moves the tracker attached on the surgical instrument. Using the tracking system comprising the trackers 6 and the aforementioned lighthouses 12, the position of the surgical instrument 3 may be tracked in the anatomical model as the user moves the trackers attached on the surgical instruments. Preferably, this system may allow for haptic feedback, meaning that there is a synchronization in 3D space of the real world models with the virtual world models. The anatomical model mounted on the cart, the surgical instruments, and optionally shoulder joint plastic bones inside the plastic shoulder model 1, have corresponding 3D virtual models, i.e. virtual representations, in the virtual or computer environment i.e. in the virtual model displayed on the monitor/display. As mentioned further above, preferably there is haptic feedback, so that every time the user hits an anatomic structure with an instrument in the virtual world (visible collision on a monitor or 3D world with the headset) there is an actual collision as well of the real instrument on the plastic model, for example, hitting the head of the humerus with a grasper.

It is noted that in real surgery, the arthroscope is typically put into the knee/shoulder or any other joint through small incisions. Typically, once the arthroscope is inserted, a lens system, that is typically comprised by the arthroscope, magnifies the inside of the joint by gathering light and focusing that light to form a real image that is viewed on a TV monitor. In a preferred embodiment of the present invention, said embodiment comprising an arthroscope as the one described further above, the aforementioned rotation of the lens shift is performed using the lens offset of a virtual camera that is comprised by the second virtual model. A virtual camera can be considered as being a simulated device through which the user of the simulator views the simulated world in the computer.

In a preferred embodiment of the present invention, the at least one surgical tool comprises an inertial measurement unit (IMU), also called first IMU, which is an electronic component, and is used to calculate the orientation of a front part of the arthroscope relative to the 3D space. More specifically, the IMU is used or is configured to calculate rotation of the surgical tool's part (the part that is insertable through the anatomical model's aperture) with respect to the handle of the surgical instrument. Preferably said part is a front part of the surgical instrument, and typically is a shaft. The tracking sensor is preferably mounted or attached to/on the handle or rear part of the surgical instrument. The rotation of the shaft-front part of the surgical instrument can be done by calculating relative angles. The tracking sensor on the surgical instrument also has a built-in IMU which can be called second IMU. Advantageously, the relative position of the first IMU to the position of the second IMU may provide the absolute rotation of the handle of the rear part of the instrument to the front part -shaft of the instrument.

This orientation that is calculated from the first and second IMUs is an absolute value that can be used by the computer system e.g. by a computer program stored and executed on said computer system, for rotation or displacement of the lens in the virtual camera. The lens shift may offset the tracking sensor based on the orientation value obtained from the first and second IMUs. Preferably, the lens shift may offset the virtual camera by 30 or 70 degrees or any value and allow for the simulation of the function of a rotating viewing field of a real arthroscope or endoscope. Advantageously, this may allow simulation of the field of view that is experienced by the user in actual or real arthroscopy or endoscopy. Field of view refers to the viewing angle encompassed by the lens and may vary according to the type of arthroscope. Wider viewing angles make orientation by the observer much easier. Rotation of the forward oblique viewing (30- and 70-degree) arthroscopes allows a much larger area of the surgical field to be observed.

In a preferred embodiment, the surgery simulation system comprises the aforementioned tracking system which comprises the tracking sensors which are mounted on the anatomical model and or the surgical tools, and also comprises lighthouse boxes (i.e. lighthouse stations), and the tracking system works as follows. The lighthouse stations are passive, and they just need power to work. There is no radio signal between the lighthouse boxes and the tracking sensors or pc. However, the lighthouse stations can communicate via radio signals for synchronization purposes. The lighthouse boxes work similarly to lighthouses in maritime navigation: they send out infrared light signals which can be detected by IR-diodes of the tracking sensors. In an example, the lighthouse boxes first they send an omnidirectional flash.

This flash is simultaneously from the lighthouse stations and when detected by the tracking sensors act as a "start now to trigger a stopwatch"-command. Then each station transmits two IR-laser swipes consecutively - much like a 'scanning line' through the area where the tracking sensors are located. One swipe is sent horizontally, the other one after that is transmitted vertically. The IR-Diodes register the laser swipes on different times due to the speed of the angular motion of the swipe. With the help of the (tiny) time differences between the flash and the swipes and also because of the fixed and know position of the IR-diodes on a case of the tracking system, the exact position and orientation of the tracking sensors can be calculated.

The calculated position/orientations are sent to the computer system along with any other position relevant sensory data. Some advantages of using the aforementioned tracking system are the following. The components of the lighthouse boxes are simple and cheap.

They lighthouse boxes don't need a high bandwidth connection to any of the surgery simulation system's components (e.g. to the computer). The position/orientation calculations are fast and can be easily handled by simple CPUs/micro controllers. No image processing CPU (central processing unit) time is consumed like on camera-based solutions.

Nevertheless, commercially available tracking systems typically have a 3 cm positional error, as developed by the manufacturer. This tracking positional error makes it unsuitable for mm of movement accuracy needed in endoscopy/arthroscopy. The surgery simulation system of the present invention, eliminates or reduces greatly that error and makes the system suitable for arthroscopy and endoscopy use. The use of the second virtual reality system of the present invention, increases positional accuracy of the surgical instruments in 3D (three-dimensional) space. The is achieved with the use of the proximity sensors, and does not require modifying the already commercially available tracking sensors or tracking systems which can be comprised by the surgery simulation system of the present invention. The proximity sensors are located in the shoulder model and allow for accurate placement of the surgical instruments in the shoulder model through the holes of the model. There are used for snapping processes, i.e. for detecting when the surgical tool enters or touches (i.e. snaps) the aperture (hole) of the anatomical model. This process allows for millimeter (mm) (e.g. 3 mm) of accuracy in tracking of the surgical instruments within the anatomical model. The shoulder model can be a plastic shoulder model.

The anatomical model of the embodiment shown in FIG. 1-4 is a shoulder model comprising apertures (holes) 2. Said shoulder model is also shown in FIG. 5 wherein there is also seen an adaptor (slot) 13 on which one of the tracking sensors 6 can be adapted or attached on/to the anatomical model 1 for tracking the anatomical model's location. The surgical instruments are inserted in the shoulder model through the holes 2. These holes have proximity sensors 7 which are snapping sensors. These proximity sensors may be capacitive, or inductive, optical or of any other type that can detect when the tip of the surgical instrument is in the hole. These snapping sensors can be used for defining the moment of proximity of the tip of the instrument to the surface of the hole. At that moment, the computer system starts defining the second coordinates of the inserted surgical tool. In addition, preferably the proximity sensor triggers the computer system to track the inserted surgical instrument only around a fixed point -the point of insertion. This may allow for simulation of the pivot and piston movement that is typically seen in arthroscopy or endoscopic surgery. Hence, the advancement or pull back of the surgical instrument in the hole or surgery portal in different directions or positions, may be detected. Advantageously, this can improve tracking because the tracker is forced to track only the 3D rotation around a pivot point, and also track the movement along one axis which is the axis of piston motion of the instrument through the hole of the plastic model. Hence, advantageously the accuracy of the tracking can be improved in the range of very few millimeters, making the system suitable for endoscopy or arthroscopy simulation.

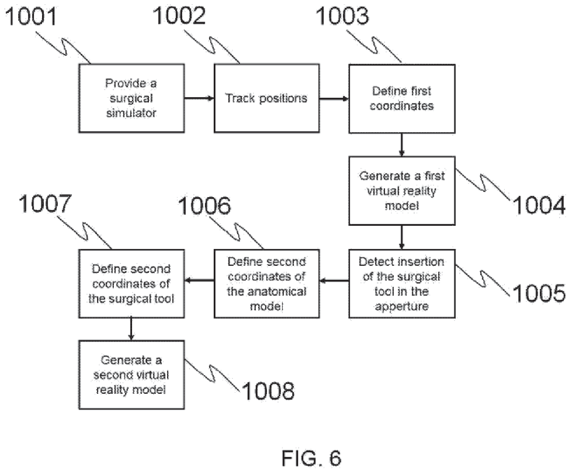

FIG. 6 shows a flow diagram of a preferred embodiment of a method according to the second aspect of the invention, wherein the method comprises the following steps:
Step 1001: providing an anatomical model with one or more apertures, at least one surgical tool that comprises a part which is movable though the one or more apertures, a first virtual reality system that comprises the computer and a set of tracking sensors on the anatomical model and on the surgical tool, and a second virtual reality system that comprises the computer system and one or more proximity sensors which are located at the one or more apertures, the tracking sensors being connected to the computer system;
Step 1002: with the tracking sensors tracking respective positions of the anatomical model and of the surgical tool;
Step 1003: with the computer system defining first coordinates of the anatomical model and of the at least one surgical tool according to their respective tracked positions;
Step 1004: with the computer generating a first virtual reality model according to the first coordinates, the first virtual reality model comprising virtual representations of the anatomical model and of the at least one surgical tool;
Step 1005: with the one or more proximity sensors detecting an insertion of the part of the at least one surgical tool at the respective aperture where the proximity sensor is located;
Step 1006: with the computer system defining second coordinates of the anatomical model;
Step 1007: with the computer system defining second coordinates of the inserted surgical tool as a function of the second coordinates of the anatomical model;
Step 1008: with the computer system generating a second virtual reality model according to the second coordinates, the second virtual reality model comprising respective virtual representations of the anatomical model and of the inserted surgical tool.

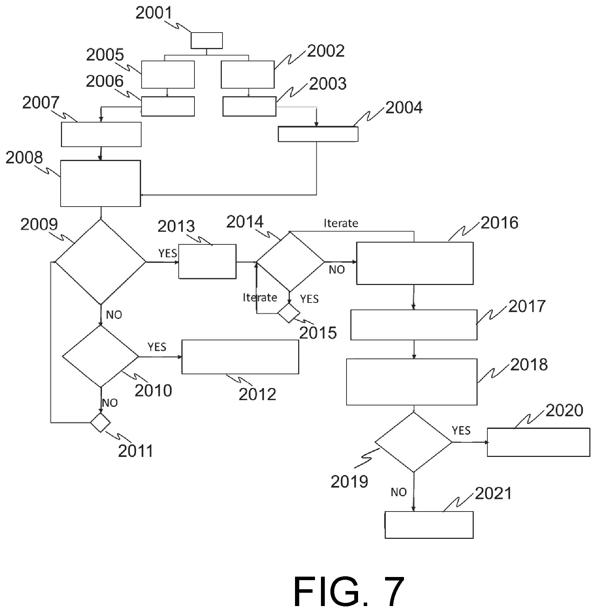

FIG. 7 shows an algorithm or computer implemented method executed by the computer system of a preferred embodiment of the present invention. The computer system starts 2001 executing the algorithm and receives 2002 signals from the tracking sensors from which it continuously receives 2003 the transformation in three-dimensional space. Accordingly, the computer system creates 2004 and array of n objects (instruments) driven by n trackers. The computers system also receives 2005 signals from the proximity sensors on the anatomical model, and continuously receives 2006 the flow of current from k proximity sensors. In step 2007 the computer (i.e. the computer system) records the flow of current inside an array of k objects (**holes**) representing each individual proximity sensor located at the surface of each physical/virtual hole, and on start, hold the initial flow of current of each hole (**startingSensorValues**) inside an array. In step 2008 the computer declares an instance of holes and instance of **instruments**; declares an array of **activeHoles** (the holes currently in use); and declares 2 sets of integers (collection of unique elements) of **uniqueindexHoles** {}, **uniqueIndexInstruments** { } representing the indices of the holes and the indices of the instruments. Step 2009 represents a question posed which is: "Does one of the holes has reduced its flow of current?" which can be expressed as "holes[index] < startingSensorValues[index]?". If the answer is NO then in step 2010 check if instruments are outside the holes. The corresponding question 2010 can be expressed as "index ∈ uniqueIndexHoles ?" and "(uniqueIndexHoles contains index?)". If the answer to question 2010 is YES, then computer system proceeds to clean up ("**uniqueIndexHoles** remove this element index" and "uniqueIndexInstruments remove current instrument index (activeHoles[index]") in step 2012. If the answer to the question 2010 is NO then return 2011 to step 2009. If the answer to the question of step 2009 is YES then this means that at least one of the instruments has been inserted in a hole, and the next step in the algorithm is step 2013 where the computer system finds out which of the currently moving outside the physical model instruments is at closest distance from the touched sensor / hole, and declares a dictionary (key, value) ("**instrumentIndicesAndDistances"**). Next step 2014 represents the logical question of whether **uniqueInstruments** contains **index**, and said question can be expressed as: "**index** ∈ **uniqueInstruments**?". If the answer is YES then continue to step 2015 and iterate to step 2014. If the answer in NO move to step 2016 wherein in **instrumentIndicesAndDistances** dictionary add "key: **instruments[index]**", and "value: sqrMagnitude(**instruments[index]**.position - **holes[index].**position)". Then iterate to step 2014 or close loop and proceed to step 2017 where the computer system sorts **instrumentIndicesAndDistances** in ascending order, which can be expressed as "**instrumentIndicesAndDistances[0]** => (**instruments[index]**, min distance)". Then in step 2018 create **visibleInstrument** that is an identical instrument copy of **instruments[index]**, its tip pointing at the surface of the closest to the touched hole/ sensor. Also, in step 2018 the following are being executed: hide the visibility of the currently used instrument **instruments[index]**; lock the x, z position axes to zero of the **visibleInstrumen**t; **visibleInstrument** mimic the rotation of the **instruments[index];** and **activeHoles[index] = true** (this hole/ sensor index is busy). Hence, it is to be understood that in step 2018 a new (virtual) instrument has just been "born" when the outside of the model moving instrument has touched a sensor. This new instrument will be alive while the user is moving it inside the model.

Once outside the model, this instrument will be destroyed and, in its position, the previous (virtual) instrument will appear again. Step 2019 represents the question: "Is **instruments[index]** moving inside model?". If the answer is YES, then in step 2020, the computer system makes **visibleInstrument** moving in the y direction with (i.e utilizing) the dot product of the difference between **instruments[index] -holes[index].** This can be expressed as: "**visibleInstrument**.position = (0, dot product, 0)". If the answer to question 2019 is NO, then in step 2021 destroy **visibleInstrument** and make **instruments[index]** visible again.

Various modifications and additions can be made to the embodiments discussed without departing from the scope of the invention. For example, while the embodiments described above refer to particular features, the scope of this invention also includes embodiments having different combination of features and embodiments that do not include all of the above-described features. Thus, the present invention is not intended to be limited to the embodiments shown herein but is to be accorded the widest scope consistent with the principles and novel features disclosed herein.

## Claims

1. A surgery simulation system configured for use with tracking sensors, comprising:
an anatomical model (1) having one or more apertures (2) and configured to receive a tracking sensor (6);
at least one surgical tool (3) comprising a part (4) movable through an aperture (2) and configured to receive a tracking sensor (6);
one or more proximity sensors (7), each located at a respective aperture (2) and configured to detect insertion of the part (4) at that aperture; and
a computer system (5) configured to be operatively connected to the tracking sensors, or a tracking system comprising them, and to the proximity sensors, and configured to:
(a) define first coordinates of the anatomical model and surgical tool according to their tracked positions and generate a first virtual reality model comprising respective virtual representations of the anatomical model and surgical tool according to the first coordinates;
(b) in response to detection of insertion, define second coordinates of the anatomical model as coordinates of a reference point at the aperture, fixed during generation of a second virtual reality model, and define second coordinates of the inserted surgical tool as a function of the fixed coordinates and the first coordinates of that tool; and
(c) generate the second virtual reality model comprising respective virtual representations of the anatomical model and inserted surgical tool according to the second coordinates, including calculating a pivot angle about the reference point and a displacement along an axis of piston movement through the aperture;
**characterised in that** the surgical tool is configured to receive its tracking sensor at the end opposite the end that first enters the aperture, and the computer system is configured to define the second coordinates of the inserted surgical tool also as a function of its length, by using, at the start of insertion detected by the proximity sensor,
the information that the distance between that tracking sensor and the aperture equals the tool length to define initial values of the second coordinates, and using that information to define subsequent values of the second coordinates during movement of the tool through the aperture.

2. The surgery simulation system according to claim 1, wherein the computer system continues defining and storing the first coordinates in a memory while defining the second coordinates.

3. The surgery simulation system according to claim 1, wherein the fixed coordinates are x = 0, y = 0 and z = 0, and the second coordinates of the inserted surgical tool are defined with respect to those zero coordinates.

4. The surgery simulation system according to claim 1, further comprising a virtual reality tracking system with the tracking sensors mounted on the anatomical model and surgical tool and operatively connected to the computer system.

5. The surgery simulation system according to claim 4, wherein the tracking system comprises beacons emitting electromagnetic signals other than optical signals, the tracking sensors receive those signals, and the received signals are processed and compared by the tracking system to identify the positions of the tracking sensors relative to the beacons.

6. The surgery simulation system according to claim 4, wherein the tracking system comprises beacons emitting optical signals, the tracking sensors receive those signals, and the received signals are processed and compared by the tracking system to identify the positions of the tracking sensors relative to the beacons.

7. The surgery simulation system according to claim 4, wherein the surgical tool comprises a first inertial measurement unit at a front part or shaft, its tracking sensor comprises a second inertial measurement unit and is mounted on a rear part of the tool, the two units measure respective rotations of the surgical tool and tracking sensor and emit corresponding measurement signals to the computer system, and the computer system receives both measurement signals and calculates a rotation and/or displacement of the surgical tool from those signals.

8. The surgery simulation system according to claim 1, further comprising a display (8), wherein the computer system renders the first virtual reality model on the display and, when defining the second coordinates, stops rendering the first model and starts rendering the second virtual reality model on the display.

9. A computer-implemented method of simulating surgery using an anatomical model (1) having one or more apertures (2), at least one surgical tool (3) with a part (4) movable through an aperture, tracking sensors (6) on the anatomical model and surgical tool, and a proximity sensor (7) at the aperture, comprising, by a computer system (5) operatively connected to those sensors:
(a) receiving tracking data, defining first coordinates of the anatomical model and surgical tool according to their tracked positions, and generating a first virtual reality model comprising respective virtual representations of the anatomical model and surgical tool according to those coordinates;
(b) in response to a proximity-sensor signal detecting insertion, defining second coordinates of the anatomical model as coordinates of a reference point at the aperture, fixed during generation of a second virtual reality model, and defining second coordinates of the inserted surgical tool as a function of those fixed coordinates and the first coordinates of that tool; and
(c) generating the second virtual reality model comprising respective virtual representations of the anatomical model and inserted surgical tool according to the second coordinates, including calculating a pivot angle about the reference point and a displacement along an axis of piston movement through the aperture;
**characterised in that** the tool's tracking sensor is at the end opposite the end that first enters the aperture, and the method further comprises defining the second coordinates of the inserted surgical tool also as a function of its length, by using, at the start of insertion detected by the proximity sensor, the information that the distance between that tracking sensor and the aperture equals the tool length to define initial values of the second coordinates, and using that information to define subsequent values during movement through the aperture.

10. The method according to claim 9, wherein the surgical tool comprises a first inertial measurement unit at a front part or shaft and its tracking sensor comprises a second inertial measurement unit and is mounted on a rear part of the tool, and the method comprises receiving, at the computer system, signals from both units measuring respective rotations of the surgical tool and tracking sensor, and calculating a rotation and/or displacement of the surgical tool from those signals.

11. A surgery simulation system configured for use with a set of tracking sensors for tracking respective positions of an anatomical model and at least one surgical tool, the surgery simulation system comprising:
an anatomical model having one or more apertures and configured to receive a tracking sensor;
at least one surgical tool that comprises a part movable through an aperture of the anatomical model and is configured to receive a tracking sensor on a rear part of the surgical tool;
a computer system configured to be operatively connected to the tracking sensors or to a tracking system comprising them, so as to form a first virtual reality system upon such connection, and to define first coordinates of the anatomical model and the at least one surgical tool according to their respective tracked positions; and
a second virtual reality system comprising the computer system and one or more proximity sensors located at respective apertures and configured to detect insertion of the part of a surgical tool at the respective aperture, the computer system being configured, in response to detection of insertion, to define second coordinates of the anatomical model and the inserted surgical tool, the second coordinates of the inserted surgical tool being a function of the second coordinates of the anatomical model;
wherein the computer system is configured to generate a first virtual reality model according to the first coordinates and, when defining the second coordinates, a second virtual reality model according to the second coordinates, the first virtual reality model comprising respective virtual representations of the anatomical model and the at least one surgical tool, and the second virtual reality model comprising respective virtual representations of the anatomical model and the inserted surgical tool;
wherein the surgical tool comprises a first inertial measurement unit at a front part or shaft of the surgical tool, configured to measure rotation of the surgical tool and emit corresponding measurement signals to the computer system; and
wherein the computer system is configured to receive said measurement signals and measurement signals from a second inertial measurement unit comprised in the tracking sensor receivable on the rear part of the surgical tool, the latter signals representing rotation of that tracking sensor, and to calculate a rotation and/or displacement of the surgical tool using the measurement signals from both inertial measurement units.

12. The surgery simulation system according to claim 11, wherein the computer system is configured to define the second coordinates of the inserted surgical tool as a function of both the second coordinates of the anatomical model and the first coordinates of the inserted surgical tool.

13. The surgery simulation system according to claim 12, wherein the second coordinates of the anatomical model are coordinates of a reference point at the aperture in which the surgical tool is inserted and remain fixed during generation of the second virtual reality model.

14. The surgery simulation system according to claim 13, wherein, for generating the second virtual reality model, the computer system is configured to calculate a pivot angle of the inserted surgical tool about the reference point and a displacement of the inserted surgical tool along an axis of piston movement through the aperture.

15. The surgery simulation system according to claim 14, wherein the surgical tool is configured to receive its tracking sensor at the end opposite the end that first enters the aperture, and the computer system is configured to define the second coordinates of the inserted surgical tool also as a function of its length, by using, at the start of insertion detected by the proximity sensor, the information that the distance between that tracking sensor and the aperture equals the tool length to define initial values of the second coordinates, and using that information to define subsequent values of the second coordinates during movement of the tool through the aperture.
